# EUROPEAN PATENT APPLICATION

(11) **EP 4 682 196 A1**
(43) Date of publication of application: **21.01.2026**
(21) Application number: 23936013.4
(22) Date of filing: 09.05.2023
(51) Int. Cl.: C08J 11/26, C07C 69/96

(54) **POLYCARBONATE OLIGOMER HAVING UNSATURATED DOUBLE BONDS, PREPARATION METHOD THEREFOR, CURABLE COMPOSITION, AND PREPARATION METHOD FOR LOW-DIELECTRIC CURED PRODUCT**

(71) Applicant: Swancor Innovation & Incubation Co., Ltd., Nantou City, Nantou County 540028 (TW)
(72) Inventor: HUANG, Yun-wen, Nantou County Taiwan 540028 (CN); WANG, Meng-wei, Nantou County Taiwan 540028 (CN); LAI, Pei-cheng, Nantou County Taiwan 540028 (CN)
(74) Representative: Ter Meer Steinmeister & Partner
(86) International application number: PCT/CN2023/092929
(87) International publication number: WO 2024/229685

(57) **Abstract**

A polycarbonate oligomer having unsaturated double bonds. The polycarbonate oligomer has a structure as represented by formula (I). The structure does not contain a high-polarity alcohol group, can be cured by means of a free radical polymerization reaction, and has application potential in products such as a high-frequency circuit board. In addition, the polycarbonate oligomer can be copolymerized with a currently widely used polyphenylene ether resin, and the cured product can have good electrical characteristics and heat resistance, which can provide a balanced solution for developing a high-order material and environmental protection.

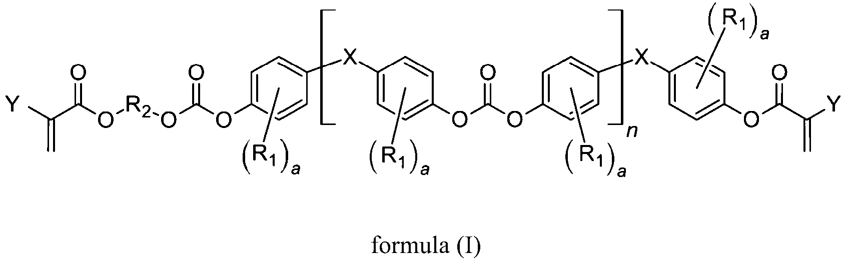

## Description

### BACKGROUND

### Field of Invention

The present disclosure relates to a polycarbonate oligomer with unsaturated double bonds, a manufacturing method thereof, a curable composition and a manufacturing method of a cured product with low dielectric property. More particularly, the present disclosure relates to a polycarbonate oligomer with unsaturated double bonds that can be copolymerized with a polyphenylene ether resin, manufacturing method thereof, a curable composition and a manufacturing method of cured product with low dielectric property.

### Description of Related Art

Polycarbonate (PC) is a thermoplastic polymer formed by the transesterification of bisphenol A and diphenyl carbonate at high temperatures. Polycarbonate exhibits excellent heat resistance and mechanical strength and is widely used in consumer products such as eyeglass lenses, optical discs, data storage devices, and automotive headlights.

With the increasing use of polycarbonate, the issue of polycarbonate recycling has gradually attracted attention. Currently, the recycling of waste polycarbonate products in the market is mostly carried out through physical processing. This involves separating the polycarbonate from the metal components in the product, then crushing, mixing, and subjecting it to high-temperature processing to produce other products. However, the crushing process reduces the molecular weight of the polycarbonate, which in turn deteriorates the mechanical properties and other physical properties of the recycled product, thus significantly limiting its application.

Currently, many studies are attempting to chemically recycle waste polycarbonate products, most of which involve degradation to obtain bisphenol A (BPA) monomer for reuse in polycarbonate synthesis. However, this method requires large amounts of solvents or catalysts and cumbersome purification steps to obtain high-purity BPA. This approach is difficult to commercialize due to environmental and cost considerations.

In recent years, the demand for consumer electronics has increased significantly, and the demand for circuit boards has also risen. For example, Japan has a precedent for using polycarbonate in electronic materials (such as publication number TW 202024175A). The aforementioned patent uses polycarbonate oligomers as epoxy hardeners. After copolymerization, the two can form an epoxy resin cured product with good heat resistance and electrical properties. The measured dielectric constant (Dk) of this material is 2.68 and the dielectric loss tangent (Df) is 0.0132, making it a potential material for printed circuit boards.

In response to the high frequency and high speed requirements of current high-end circuit boards, NORYLTM SA9000 resin launched by SABIC has gradually become mainstream choice. It is a polyphenylene ether (PPE) oligomer containing a difunctional unsaturated group at one end. After free radical polymerization, it has good heat resistance and rigidity. Because it does not produce polar functional groups after curing, it has a relatively lower dielectric loss tangent and dielectric constant than traditional epoxy resins.

**In** light of the growing environmental awareness in various countries, the recycling and remanufacturing of plastics have become increasingly important. The European Union has also imposed a ban on certain plastic products, requiring that a certain percentage of recycled plastic be used in their production. In response to this trend, such thinking has gradually spread to various chemical products, and brand manufacturers proactively implement restrictive planning at the raw material sourcing. However, at present, the materials used in high-end circuit board products are difficult to manufacture using recycled plastic. Additionally, due to the product's requirements for electrical properties, free radical polymerization curing systems have gradually become the mainstream. Based on the above, if waste polycarbonate can be modified into short-chain unsaturated resins, it may retain good electrical properties and heat resistance, and can be used as one of the recycled raw materials in circuit board materials to align with future trends.

### SUMMARY

The purpose of the present disclosure is to provide a method for recycling and preparing oligomers from waste polycarbonate. The prepared oligomers have good copolymerization operability with polyphenylene ether materials widely used in high-frequency circuit boards on the market today, and have excellent heat resistance and electrical properties after curing. This can provide the industry with a high-end circuit board material obtained by high-value recycling of raw materials.

According to one embodiment of the present disclosure, a polycarbonate oligomer with unsaturated double bonds is provided. The polycarbonate oligomer with unsaturated double bonds has a structure represented by formula (I): 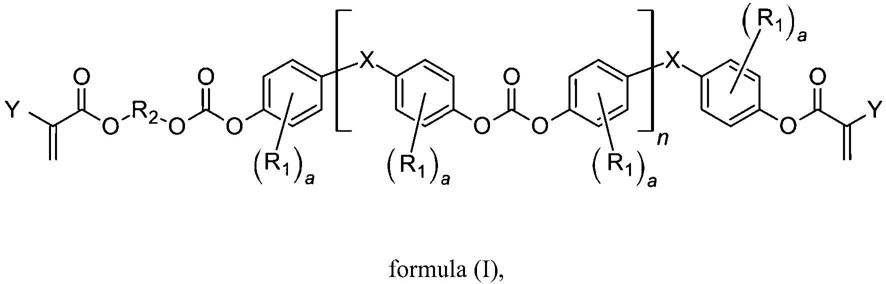 in which R₁ is a hydrogen atom, an alkyl group with a carbon number of 1 to 6, an allyl group, an alkoxy group with a carbon number of 1 to 6, an aryl group with a carbon number of 6 to 12, or a halogen atom; R₂ is an alkyl group with a carbon number of 1 to 12; X is a single bond, a cycloalkyl group with a carbon number of 3 to 12, a structure represented by formula (1), formula (2), formula (3), formula (4), formula (5), formula (6), formula (7), formula (8), or formula (9): 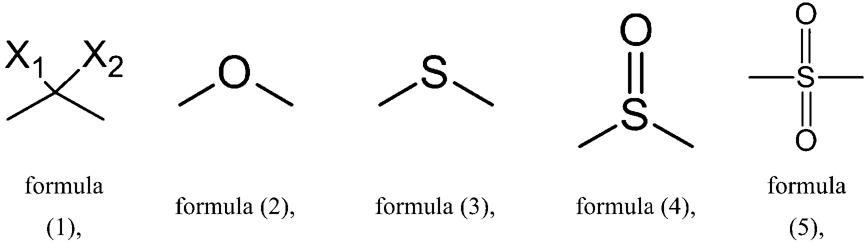 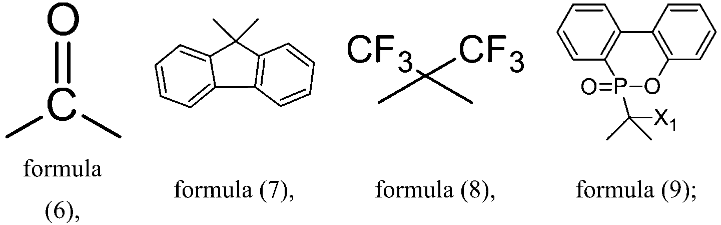 in which X₁ and X₂ are a hydrogen atom, an alkyl group with a carbon number of 1 to 6, or an aryl group with a carbon number of 6 to 12, independently; in which Y is a hydrogen atom or a methyl group, a is an integer of 0 to 4, and n is an arbitrary number of 0 to 30.

According to another embodiment of the present disclosure, a manufacturing method of the aforementioned polycarbonate oligomer with unsaturated double bonds is provided. The manufacturing method includes the steps as follows. An alcoholysis step is performed, in which polycarbonate is dissolved in a reaction solvent, and a first compound and a first catalyst are added to conduct a alcoholysis reaction to obtain a reaction solution of a polycarbonate oligomer with a one-sided unsaturated double bond, in which the first compound has a structure represented by formula (II), and the polycarbonate oligomer with a one-sided unsaturated double bond has a structure represented by formula (III): 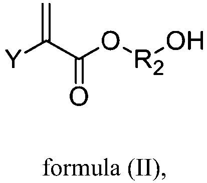 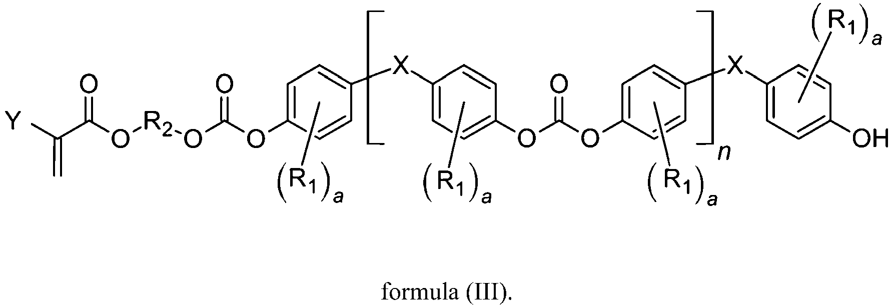 An esterification step is performed, in which a second compound and a second catalyst are added to the reaction solution to conduct an esterification reaction to obtain a product for purification, in which the second compound has a structure represented by formula (IV): 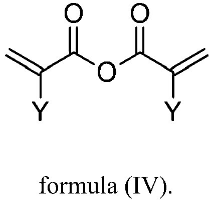 A washing step is performed, in which the product for purification is precipitated by and washed with an alcohol solvent to form a product for drying. A drying step is performed, in which the product for drying is filtered and dried to obtain the polycarbonate oligomer with unsaturated double bonds, in which R₁ is a hydrogen atom, an alkyl group with a carbon number of 1 to 6, an allyl group, an alkoxy group with a carbon number of 1 to 6, an aryl group with a carbon number of 6 to 12, or a halogen atom; R₂ is an alkyl group with a carbon number of 1 to 12; X is a single bond, a cycloalkyl group with a carbon number of 3 to 12, a structure represented by formula (1), formula (2), formula (3), formula (4), formula (5), formula (6), formula (7), formula (8), or formula (9): 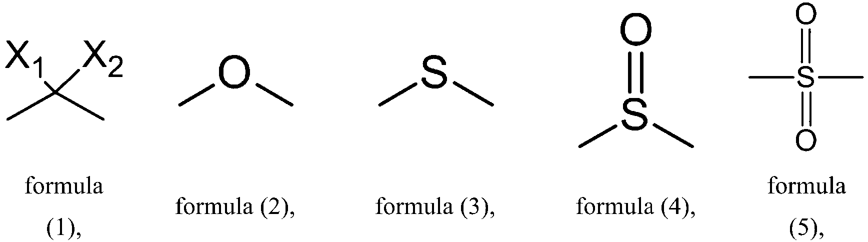 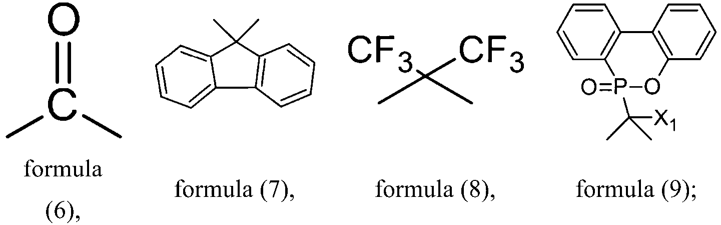 in which X₁ and X₂ are a hydrogen atom, an alkyl group with a carbon number of 1 to 6, or an aryl group with a carbon number of 6 to 12, independently; in which Y is a hydrogen atom or a methyl group, a is an integer of 0 to 4, and n is an arbitrary number of 0 to 30.

In some embodiments, a molar ratio of the polycarbonate to the first compound is 1:10 to 1:50.

In some embodiments, the reaction solvent is selected from the group consisting of N,N-dimethylacetamide, N-methylpyrrolidone, dimethylformamide, methoxybenzene, dimethyl sulfoxide, propylene glycol methyl ether acetate, propylene glycol methyl ether propionate and cyclohexanone.

In some embodiments, the first catalyst and second catalyst are respectively selected from the group consisting of 4-dimethylaminopyridine, imidazole, 2-methylimidazole, 2-phenylimidazole, 2-ethyl-4-methylimidazole, 1,8-diazabicyclo[5.4.0]undec-7-ene and 1,5,7-triazabicyclo[4.4.0]dec-5-ene.

In some embodiments, a molar ratio of the first compound to the first catalyst is 1:0.002 to 1:0.004.

In some embodiments, in the alcoholysis step, the alcoholysis reaction between the polycarbonate and the first compound is conducted at 70 °C to 180 °C.

In some embodiments, an addition amount of the first catalyst and the second catalyst is 10 weight percent to 30 weight percent of a content of the first compound.

In some embodiments, a molar ratio of the first compound to the second compound is 1:1 to 1:5.

In some embodiments, in the esterification step, the esterification reaction between the polycarbonate oligomer with the one-sided unsaturated double bond and the second compound is conducted at 70 °C to 100 °C.

According to yet another embodiment of the present disclosure, a curable composition is provided. The curable composition includes the aforementioned polycarbonate oligomer with unsaturated double bonds, a polyphenylene ether resin with unsaturated double bonds, a solvent, and a free radical initiator.

In some embodiments, a mass ratio of the polycarbonate oligomer with unsaturated double bonds to the polyphenylene ether resin with unsaturated double bonds is 0.1 to 1.

In some embodiments, the free radical initiator is selected from the group consisting of a peroxide and an azo initiator.

In some embodiments, an addition amount of the free radical initiator is 0.5 weight percent to 3.0 weight percent of a total content of the polycarbonate oligomer with unsaturated double bonds and the polyphenylene ether resin with unsaturated double bonds.

In some embodiments, the solvent is selected from the group consisting of N,N-dimethylacetamide, N-methylpyrrolidone, dimethylformamide, methoxybenzene, dimethyl sulfoxide, propylene glycol methyl ether acetate, propylene glycol methyl ether propionate, γ-butyrolactone, butanone, cyclohexanone, toluene and xylene.

According to a further embodiment of the present disclosure, a manufacturing method of a cured product with low dielectric property is provided. The manufacturing method includes the step as follows. The aforementioned curable composition is cured at a curing temperature to obtain a cured product with low dielectric property, in which the curing temperature is 140 °C to 250 °C.

Thereby, the present disclosure proposes a method for recycling and reusing waste polycarbonate, which utilizes principles such as alcoholysis and esterification to prepare bifunctional polycarbonate oligomers with unsaturated groups at their ends. These oligomers can be cured together with materials commonly used in high-frequency circuit boards, and the resulting cured products exhibit excellent electrical and physical properties, enabling waste materials to achieve the goal of high-value recycling while meeting environmental protection requirements.

### BRIEF DESCRIPTION OF THE DRAWINGS

The present disclosure can be more fully understood by reading the following detailed description of the embodiments, with reference made to the accompanying drawings.

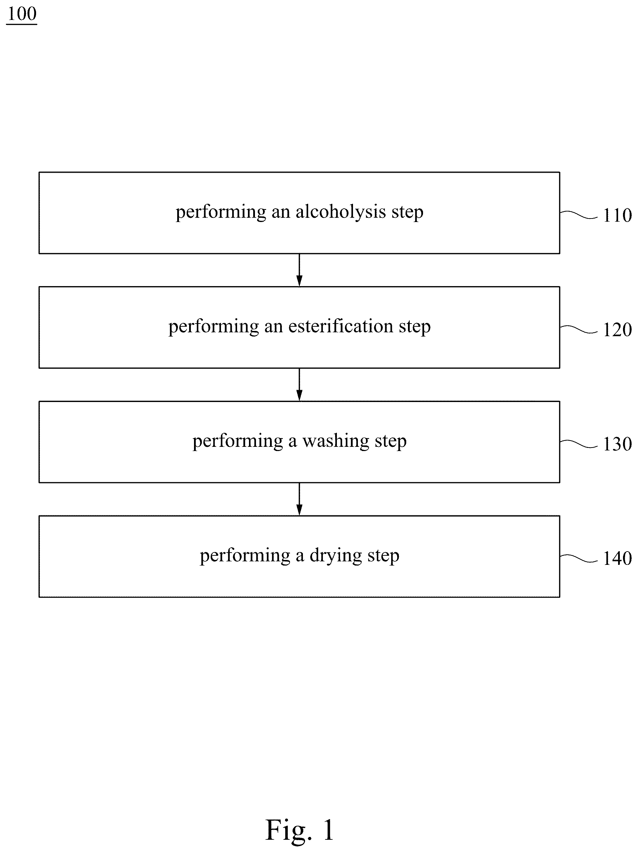

Fig. 1 is a step flow chart of a manufacturing method of a polycarbonate oligomer with unsaturated double bonds in accordance with some embodiments of the present disclosure.

### [Symbol explanation]

100: manufacturing method
110, 120, 130, 140: step

### DETAILED DESCRIPTION

The following will discuss various embodiments of the present disclosure in more detail. However, this embodiment can be an application of various inventive concepts and can be specifically implemented within a variety of specific scopes. The specific embodiments are for illustrative purposes only and are not intended to limit the scope of the disclosure.

In this disclosure, compound structures are sometimes represented using skeleton formulas. This representation may omit carbon atoms, hydrogen atoms, and carbon-hydrogen bonds. If a functional group is explicitly depicted in a structural formula, the depicted functional group shall prevail.

In this disclosure, for the sake of brevity and fluency, "a first compound having a structure represented by formula (II)" is sometimes expressed as "a first compound represented by formula (II)" or "a first compound (II)", and the representation of other compounds or groups is similar.

### < Polycarbonate oligomer with unsaturated double bonds >

According to one embodiment of the present disclosure, a polycarbonate oligomer with unsaturated double bonds is provided. The polycarbonate oligomer with unsaturated double bonds has a structure represented by formula (I): 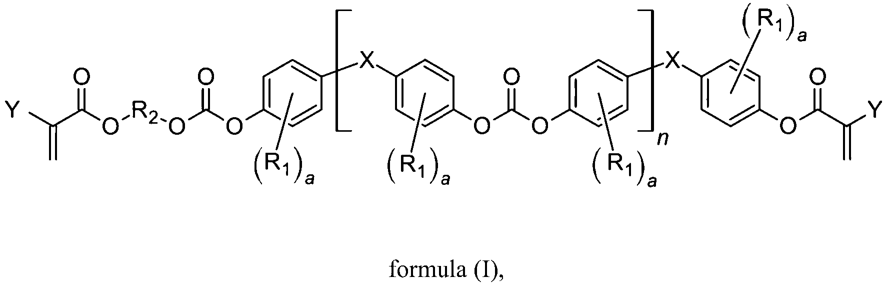 in which R₁ is a hydrogen atom, an alkyl group with a carbon number of 1 to 6, an allyl group, an alkoxy group with a carbon number of 1 to 6, an aryl group with a carbon number of 6 to 12, or a halogen atom; R₂ is an alkyl group with a carbon number of 1 to 12; X is a single bond, a cycloalkyl group with a carbon number of 3 to 12, a structure represented by formula (1), formula (2), formula (3), formula (4), formula (5), formula (6), formula (7), formula (8), or formula (9): 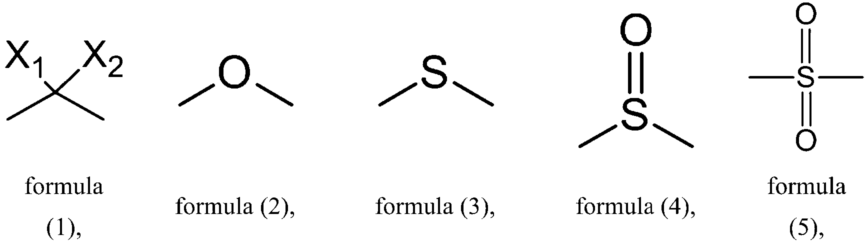 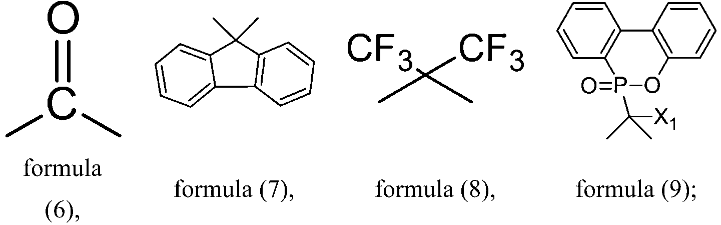 in which X₁ and X₂ are a hydrogen atom, an alkyl group with a carbon number of 1 to 6, or an aryl group with a carbon number of 6 to 12, independently; in which Y is a hydrogen atom or a methyl group, a is an integer of 0 to 4, and n is an arbitrary number of 0 to 30, in which n represents a degree of polymerization.

Accordingly, the aforementioned polycarbonate oligomer with unsaturated double bonds does not contain highly polar alcohol groups in its structure and can be cured through free radical polymerization reactions, demonstrating potential applications in products such as high-frequency circuit boards. The polycarbonate oligomer with unsaturated double bonds can be copolymerized with currently widely used polyphenylene ether resins, and the cured products exhibit excellent electrical properties and heat resistance.

### <Manufacturing method of polycarbonate oligomer with unsaturated double bonds>

Please refer to Fig. 1, which is a step flow chart of a manufacturing method 100 of a polycarbonate oligomer with unsaturated double bonds of the present disclosure. According to another embodiment of the present disclosure, a manufacturing method 100 of the above polycarbonate oligomer with unsaturated double bonds is provided. The manufacturing method 100 includes step 110, step 120, step 130, and step 140.

Step 110 is a performing an alcoholysis step, in which a polycarbonate is dissolved in a reaction solvent, and a first compound and a first catalyst are added to conduct a alcoholysis reaction to obtain a reaction solution of a polycarbonate oligomer with a one-sided unsaturated double bond, in which the first compound has a structure represented by formula (II), and the polycarbonate oligomer with a one-sided unsaturated double bond has a structure represented by formula (III):

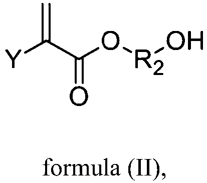

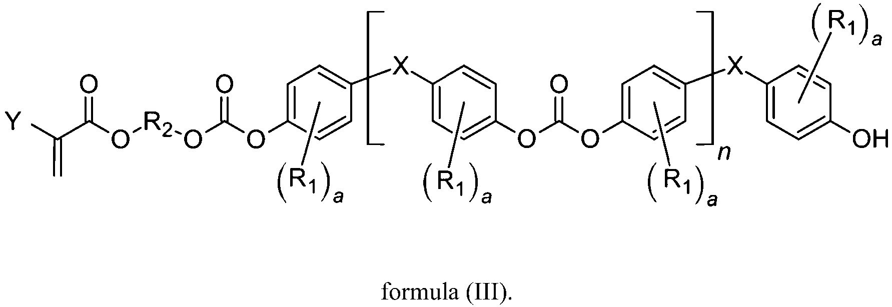

Step 120 is a performing an esterification step, in which a second compound and a second catalyst are added to the reaction solution to conduct an esterification reaction to obtain a product for purification, in which the second compound has a structure represented by formula (IV):

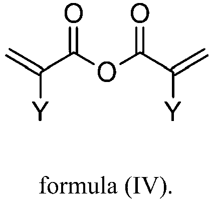

Step 130 is a performing a washing step, in which the product for purification is precipitated by and washed with an alcohol solvent to form a product for drying.

Step 140 is a performing a drying step, in which the product for drying is filtered and dried to obtain the polycarbonate oligomer with unsaturated double bonds. R₁, R₂, X, X₁, X₂, Y, a and n in formula (II), formula (III) and formula (IV) are as defined in the preceding paragraphs and are not further described here.

The aforementioned reaction solvent is selected from the group consisting of N,N-dimethylacetamide (dimethylacetamide; DMAc), N-methylpyrrolidone (N-methyl-2-pyrrolidone; NMP), dimethylformamide (DMF), methoxybenzene, dimethyl sulfoxide (DMSO), propylene glycol methyl ether acetate, propylene glycol methyl ether propionate and cyclohexanone.

The aforementioned first catalyst and the aforementioned second catalyst are respectively selected from the group consisting of 4-dimethylaminopyridine, imidazole, 2-methylimidazole, 2-phenylimidazole, 2-ethyl-4-methylimidazole, 1,8-diazabicyclo[5.4.0]undec-7-ene (DBU), and 1,5,7-triazabicyclo[4.4.0]dec-5-ene (TBU).

A molar ratio of the polycarbonate to the first compound is 1:10 to 1:50; 1:20 to 1:40 is preferred. A molar ratio of the first compound to the first catalyst is 1:0.002 to 1:0.004; 1:0.002 to 1:0.003 is preferred. An addition amount of the first catalyst and the second catalyst is 10 weight percent to 30 weight percent of a content of the first compound; 20 weight percent to 25 weight percent is preferred. A molar ratio of the first compound to the second compound is 1:1 to 1:5; 1:2.2 to 1:4.4 is preferred.

The aforementioned alcoholysis reaction and the aforementioned esterification reaction may both be carried out in a heating manner. In the alcoholysis step, the alcoholysis reaction between the polycarbonate and the first compound is conducted at 70 °C to 180 °C; the alcoholysis reaction is preferably conducted at 130 °C to 150 °C. In the esterification step, the esterification reaction between the polycarbonate oligomer with the one-sided unsaturated double bond and the second compound is conducted at 70 °C to 100 °C; the esterification reaction is preferably conducted at 70 °C to 90 °C.

For example, when in the aforementioned polycarbonate oligomer with the one-sided unsaturated double bond represented by formula (III), X is a structure represent by formula (1), X₁, X₂, and Y are methyl groups, R₁ is a hydrogen atom, and R₂ is an ethylene group, the prepared polycarbonate oligomer with unsaturated double bonds has a structure as shown in formula (I-a), and the polycarbonate oligomer with the one-sided unsaturated double bond has a structure represented by formula (III-a):

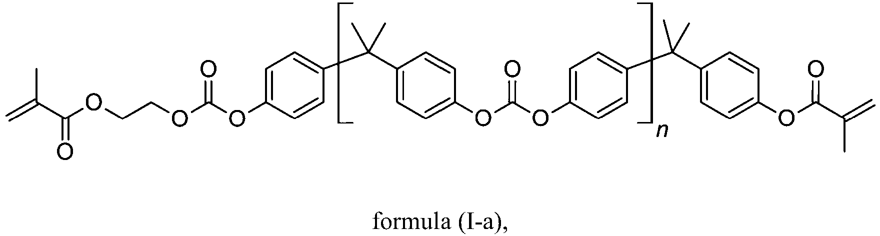

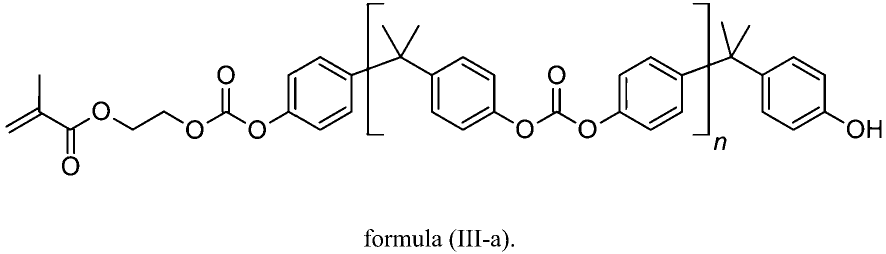

### <Curable composition>

According to yet another embodiment of the present disclosure, a curable composition is provided. The curable composition includes the aforementioned polycarbonate oligomer with unsaturated double bonds, a polyphenylene ether resin with unsaturated double bonds, a solvent, and a free radical initiator. The curable composition may undergo a curing reaction at a high temperature to obtain a cured product with low dielectric property. A mixing temperature of the curable composition may be 50 °C to 100 °C; 70 °C to 90 °C is preferred.

The free radical initiator is selected from the group consisting of a peroxide and an azo initiator. The solvent is selected from the group consisting of N,N-dimethylacetamide, N-methylpyrrolidone, dimethylformamide, methoxybenzene, dimethyl sulfoxide, propylene glycol methyl ether acetate, propylene glycol methyl ether propionate, γ-butyrolactone, butanone, cyclohexanone, toluene and xylene.

A mass ratio of the polycarbonate oligomer with unsaturated double bonds to the polyphenylene ether resin with unsaturated double bonds is 0.1 to 1. An addition amount of the free radical initiator is 0.5 weight percent to 3.0 weight percent of a total content of the polycarbonate oligomer with unsaturated double bonds and the polyphenylene ether resin with unsaturated double bonds; 1 weight percent to 2.5 weight percent is preferred. If the addition amount of the free radical initiator added is too small, curing may be incomplete, resulting in a decrease in the physical properties of the curable composition. If too much free radical initiator is added, the electrical properties of the curable composition may be decreased.

### <Manufacturing method of cured product with low dielectric property>

According to a further embodiment of the present disclosure, a manufacturing method of a cured product with low dielectric property is provided. The manufacturing method includes the step as follows. The aforementioned curable composition is cured at a curing temperature to obtain a cured product with low dielectric property, in which the curing temperature is 140 °C to 250 °C; 180 °C to 220 °C is preferred, but is not limited thereto. A person skilled in the art of the present disclosure can select an appropriate curing temperature, such as 140 °C to 180 °C, 140 °C to 220 °C, 150 °C to 250 °C, etc.

The present disclosure is further illustrated by the following specific embodiments, which are intended to facilitate a person skilled in the art of the present disclosure, so that they can fully utilize and practice the present disclosure without excessive interpretation. These embodiments should not be regarded as limiting the scope of the present disclosure, but are intended to illustrate how to implement the materials and methods of the present disclosure.

### <Preparing polycarbonate oligomer with unsaturated double bonds>

### <Example 1>

100 g (4 ×10⁻³ mole, calculated as Mn) of polycarbonate ester pellets (purchased from CHIMEI corporation, product code PC-122, Mn = 24400, Mw = 45488) was completely dissolved in 150 g of DMAc at 150 °C. 10.07 g (0.082 mole) of hydroxyethyl methacrylate was then added dropwise to the solution. After mixing evenly, 0.026 g (1.69 ×10⁻⁴mole) of DBU was added. The mixture was allowed to react for 5 hours. After the reaction was completed, the temperature was lowered, the mixture was washed with methanol, filtered by suction filtration, and dried in a vacuum oven at 78 °C to obtain the compound represented by formula (III-a). The product was coded HEPC with a yield of approximately 71%. GPC analysis of the product presents Mn = 2721 and Mw = 4061.

### <Example 2>

The first half of the process was the same as in Example 1, except that the reaction mixture was not rinsed with methanol after completion. The temperature was then lowered to 70 °C, and 2.33 g (22 weight percent of the first compound (II)) of DBU and 27.81 g (0.180 mole) of methacrylic anhydride were added in sequence. The reaction was continued at 70 °C for 6 hours. After completion of the reaction, the mixture was rinsed with methanol, filtered by suction filtration, and dried in a vacuum oven at 78 °C to obtain a white powder of formula (I-a). The product was coded HAPC-1 with a yield of approximately 73.6%. GPC analysis of the product presents Mn = 2366 and Mw = 4137.

### <Example 3>

100 g (4 ×10⁻³ mole, calculated as Mn) of polycarbonate ester pellets (purchased from CHIMEI corporation, product code PC-122, Mn = 24400, Mw = 45488) was completely dissolved in 150 g of DMAc at 150n°C. 16.51 g (0.127 mole) of hydroxyethyl methacrylate was then added dropwise to the above solution. After mixing evenly, 0.038 g (2.54 ×10⁻⁴ mole) of DBU was added. The reaction was continued for 5 hours. After the reaction was completed, the temperature was lowered to 70 °C, and 3.63 g (22 weight percent of the content of the first compound (II)) of DBU and 43.03 g (0.279 mole) were added in sequence. The reaction was continued at 78 °C for 6 hours. After completion of the reaction, the mixture was washed with methanol, filtered by suction filtration, and dried in a vacuum oven at 78 °C to obtain a white powder of formula (I-a). The product was coded HAPC-1.5 with a yield of approximately 59%. GPC analysis of the product presents Mn = 2673 and Mw = 4479.

### <Example 4>

100 g (3.8 ×10⁻³ mole, calculated as Mn) of recycled polycarbonate ester pellets (purchased from CHIMEI corporation, product code GC-122B, Mn = 26099, Mw = 47827) were completely dissolved in 150 g of DMAc at 150 °C. 14.97 g (0.115 mole) of hydroxyethyl methacrylate was then added dropwise to the above solution. After mixing evenly, 0.035 g (2.30 ×10⁻⁴ mole) of DBU was added. The reaction was continued for 5 hours. After the reaction was completed, the temperature was lowered to 70 °C, and 3.29 g (22 weight percent of the content of the first compound (II)) of DBU and 39.00 g (0.253 mole) were added in sequence. The reaction was carried out at 80 °C for 6 hours. After completion of the reaction, the product was washed with methanol, filtered by suction filtration, and dried in a vacuum oven at 78 °C to obtain a gray powder of formula (I-a). The product was coded HAGC-1.5 with a yield of approximately 70.7%. GPC analysis of the product revealed Mn = 2658 and Mw = 4885.

### <Example 5>

100 g (3.8 ×10⁻³ mole, calculated as Mn) of recycled polycarbonate pellets (purchased from CHIMEI corporation, product code GC-122B, Mn = 26099, Mw = 47827) were completely dissolved in 150 g of DMAc at 150 °C. 19.91 g (0.153 mole) of hydroxyethyl methacrylate was then added dropwise to the above solution. After mixing evenly, 0.046 g (3.02 ×10⁻⁴ mole) of DBU was added and the reaction was allowed to proceed for 5 hours. After the reaction was completed, the temperature was lowered to 80 °C, and 4.38 g (22 weight percent of the content of the first compound (II)) of DBU and 51.89 g (0.337 mole) were added in sequence. The reaction was carried out at 80 °C for 6 hours. After completion of the reaction, the product was washed with methanol, filtered by suction filtration, and dried in a vacuum oven at 78 °C to obtain a gray powder of formula (I-a). The product was coded HAGC-2 with a yield of approximately 61.9%. GPC analysis of the product revealed Mn = 2257 and Mw = 4445.

### <Comparative Example 1>

100 g (4 ×10⁻³ mole, calculated as Mn) of polycarbonate ester pellets (purchased from CHIMEI corporation, product code PC-122, Mn = 24400, Mw = 45488) were dissolved in 150 g of DMAc and heated to 120 °C for dissolution. After dissolution, 7.28 g (0.0846 mol) of methacrylic acid and 0.129 g (2.47 mol) of DBU were added dropwise and allowed to react for 3 hours. After completion of the reaction, the product was washed with methanol, filtered by suction filtration, and dried in a vacuum oven at 78 °C to obtain a white powder. The product was coded MAPC. The resulting product had poor solubility in organic solvents, presumably due to free radical cross-linking during the reaction, and could not be further manipulated or analyzed.

### <Curing reaction of curable composition>

The aforementioned polycarbonate oligomers with unsaturated double bonds can be further prepared into curable compositions and undergo curing reaction. In detail, the aforementioned polycarbonate oligomers with unsaturated double bonds, polyphenylene ether resins with unsaturated double bonds, solvents, and free radical initiators can be mixed to obtain curable compositions. Through this process, the polycarbonate oligomers, the polyphenylene ether resins, the solvents, and the free radical initiators can form prepolymers containing the curable compositions, the details of which are described in the preceding paragraphs and will not be repeated here.

Then, polycarbonate oligomers with unsaturated double bonds and polyphenylene ether resins can undergo crosslinking and curing reactions under the catalysis of the free radical initiators, and the curing reaction can be completed by heating the curable compositions. Specific examples are provided below for illustration.

### <Example 6>

0.24 g of HAPC-1, a polycarbonate oligomer with unsaturated double bonds, and 0.96 g of NORYL^{™} SA9000, a polyphenylene ether resin (available from SABIC), were dissolved in 2.856 g of cyclohexanone. The mixture was heated and mixed at 80 °C. 0.024 g (2.0 weight percent of the total weight of the polycarbonate oligomer and polyphenylene ether resin) of dicumyl peroxide was added as a free radical initiator. After stirring thoroughly, a mixed prepolymer was obtained. This mixture was then placed in an 80 °C oven for 12 hours to partially remove the solvent. The subsequent curing reaction was then gradually heated from 140 °C to 220 °C, resulting in a cured product with low dielectric property.

### <Example 7>

The preparation steps of Example 7 were the same as those of Example 6, except that the amount of the polycarbonate oligomer with unsaturated double bond HAPC-1 was changed to 0.36 g, and the amount of the polyphenylene ether resin NORYL^{™} SA9000 was changed to 0.84 g.

### <Example 8>

The preparation steps of Example 8 were the same as those of Example 6, except that the amount of the polycarbonate oligomer with unsaturated double bond HAPC-1 was changed to 0.48 g, and the amount of the polyphenylene ether resin NORYL^{™} SA9000 was changed to 0.72 g.

### <Example 9>

The preparation steps of Example 9 were the same as those of Example 6, except that the amount of the polycarbonate oligomer with unsaturated double bond HAPC-1 was changed to 0.60 g, and the amount of the polyphenylene ether resin NORYL^{™} SA9000 was changed to 0.60 g.

### <Comparative Example 2>

0.24 g of polycarbonate oligomer with unsaturated double bond HEPC and 0.96 g of polyphenylene ether resin NORYL^{™} SA9000 (available from SABIC) were dissolved in 2.856 g of cyclohexanone. The mixture was heated and mixed at 80 °C. 0.024 g (2.0 weight percent of the total weight of the polycarbonate oligomer and polyphenylene ether resin) of dicumyl peroxide was added as a free radical initiator. After stirring thoroughly, a mixed prepolymer was obtained. This mixture was placed in an 80 °C oven for 12 hours to partially remove the solvent. The subsequent curing reaction was followed by gradually increasing the temperature from 140 °C to 220 °C. After curing, the product of Comparative Example 2 exhibited severe shrinkage and phase separation.

### <Comparative Example 3>

The preparation steps of Comparative Example 3 were the same as those of Comparative Example 2, except that the amount of the polycarbonate oligomer with unsaturated double bond HEPC was changed to 0.36 g, and the amount of the polyphenylene ether resin NORYL^{™} SA9000 was changed to 0.84 g. After curing, the product of Comparative Example 3 exhibited severe shrinkage and phase separation.

### <Comparative Example 4>

The preparation steps of Comparative Example 4 were the same as those of Comparative Example 2, except that the amount of the polycarbonate oligomer with unsaturated double bond HEPC was changed to 0.48 g, and the amount of the polyphenylene ether resin NORYL^{™} SA9000 was changed to 0.72 g. After curing, the product of Comparative Example 4 exhibited severe shrinkage and phase separation.

### <Comparative Example 5>

The preparation steps of Comparative Example 5 were the same as those of Comparative Example 2, except that the amount of the polycarbonate oligomer with unsaturated double bond HEPC was changed to 0.60 g, and the amount of the polyphenylene ether resin NORYL^{™} SA9000 was changed to 0.60 g. After curing, the product of Comparative Example 5 exhibited severe shrinkage and phase separation.

### < Property testing of cured products with low dielectric properties>

The glass transition temperatures (T_{g}) of the cured products with low dielectric properties obtained in Examples 6 to 9 were measured using a differential scanning calorimeter (DSC) at a heating rate of 10 °C/min. The measurement results are listed in Table 1 below.

| Table 1 | | | |
|---|---|---|---|
| Glass transition temperatures of cured products with low dielectric properties (°C) | | | |
| Example 6 | Example 7 | Example 8 | Example 9 |
| 156.56 | 143.68 | 121.80 | 119.20 |

Based on the above results, the polycarbonate oligomers with unsaturated double bonds synthesized in this disclosure indeed exhibit good reactivity with polyphenylene ether resins, and can react and cure under the aforementioned oven conditions. The two have good compatibility and film-forming properties, making them easy to process. In addition, polycarbonate oligomers with unsaturated double bonds disclosed herein can be used in combination with polyphenylene ether resins at the aforementioned ratios.

Furthermore, in terms of heat resistance, the glass transition temperatures decreases as the amount of polycarbonate oligomers with unsaturated double bond increases, but it still maintains good heat resistance, which is sufficient to meet various application requirements.

The following section will provide further electrical testing of the cured products with low dielectric properties in Examples 6 to 9 to obtain their dielectric properties. The frequency used for testing was 10 GHz. The dielectric constant (Dₖ) and dielectric loss tangent (D_{f}) obtained are listed in Table 2 below.

| Table 2 | | | | | |
|---|---|---|---|---|---|
| Dielectric properties of cured products with low dielectric properties | | | | | |
| | Example 6 | Example 7 | Example 8 | Example 9 | SA9000 cured product |
| Dₖ | 2.69 | 2.59 | 2.69 | 2.26 | 2.77 |
| D_{f} | 0.0105 | 0.0097 | 0.0098 | 0.0087 | 0.0071 |

According to the above experimental results, compared to the SA9000 cured product formed from pure polyphenylene ether resin, the cured products with low dielectric properties formed by adding the polycarbonate oligomers with unsaturated double bonds synthesized in this disclosure exhibits better a Dₖ performance, but no significant improvement in D_{f} performance. Furthermore, the above results indicate that increasing the amount of polycarbonate oligomers with unsaturated double bonds further improves the dielectric properties of the cured products with low dielectric properties, restoring D_{f} to a better performance while also achieving a better Dₖ performance than the SA9000 cured product, demonstrating the potential of the cured products with low dielectric properties disclosed herein for high-frequency applications.

In summary, the present disclosure provides a method for recycling and reusing waste polycarbonate, which is to use a monohydric compound containing double bond structure to perform an alcoholysis reaction on the polycarbonate. After the polycarbonate is prepared into oligomers, the residual terminal phenolic groups are modified to obtain polycarbonate oligomers with unsaturated double bonds, with all terminals possessing unsaturated double bonds. The polycarbonate oligomers with unsaturated double bonds are compatible with polyphenylene ether resins in organic solvents, and the two can undergo free-radical polymerization to cure and form samples. During sample preparation, the ratio of the polycarbonate oligomers with unsaturated double bonds to the polyphenylene ether resins can be adjusted to achieve the desired physical and electrical properties. The resulting cured products with low dielectric properties exhibit excellent dielectric loss tangent and dielectric constant performance, making them desirable for application in high-frequency circuit board materials.

The above examples are merely illustrative of the principles of this disclosure and are not intended to limit its scope. For example, other alcohols or polycarbonate resins can be used and similar reactions can be used to produce polycarbonate oligomers with unsaturated double bonds of varying chemical structures. Therefore, any modifications or extensions of the above examples are intended to fall within the scope of this disclosure.

## Claims

1. A polycarbonate oligomer with unsaturated double bonds, **characterized in that** the polycarbonate oligomer with unsaturated double bonds has a structure represented by formula (I): 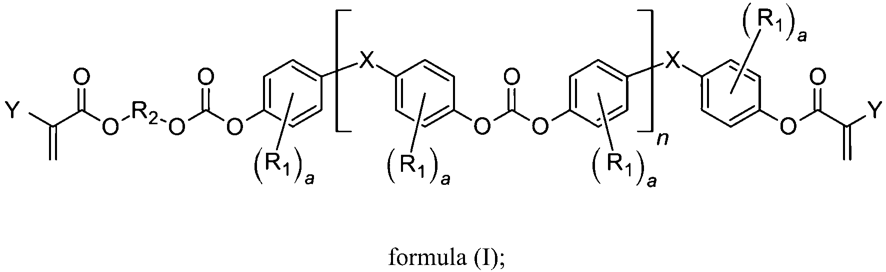
wherein R₁ is a hydrogen atom, an alkyl group with a carbon number of 1 to 6, an allyl group, an alkoxy group with a carbon number of 1 to 6, an aryl group with a carbon number of 6 to 12, or a halogen atom; R₂ is an alkyl group with a carbon number of 1 to 12; X is a single bond, a cycloalkyl group with a carbon number of 3 to 12, a structure represented by formula (1), formula (2), formula (3), formula (4), formula (5), formula (6), formula (7), formula (8), or formula (9): 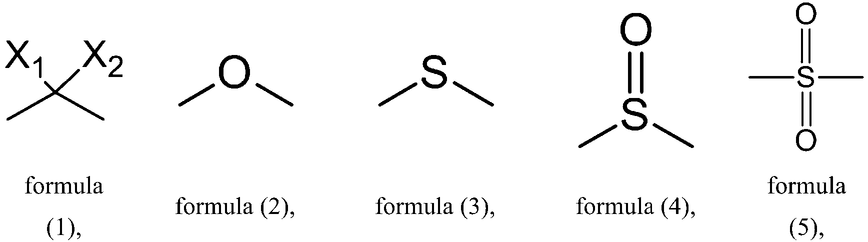 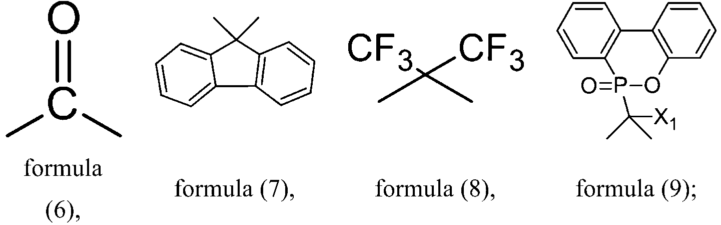
wherein X₁ and X₂ are a hydrogen atom, an alkyl group with a carbon number of 1 to 6, or an aryl group with a carbon number of 6 to 12, independently;
wherein Y is a hydrogen atom or a methyl group, a is an integer of 0 to 4, and n is an arbitrary number of 0 to 30.

2. A manufacturing method of the polycarbonate oligomer with unsaturated double bonds of claim 1, **characterized by** comprising:
performing an alcoholysis step, wherein polycarbonate is dissolved in a reaction solvent, and a first compound and a first catalyst are added to conduct a alcoholysis reaction to obtain a reaction solution of a polycarbonate oligomer with a one-sided unsaturated double bond, wherein the first compound has a structure represented by formula (II), and the polycarbonate oligomer with a one-sided unsaturated double bond has a structure represented by formula (III): 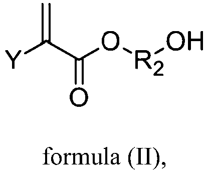 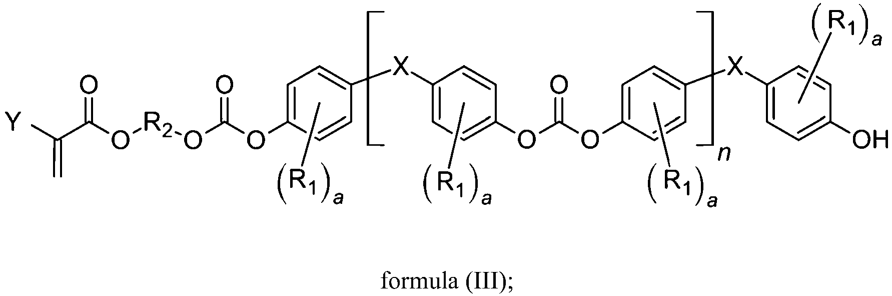
performing an esterification step, wherein a second compound and a second catalyst are added to the reaction solution to conduct a esterification reaction to obtain a product for purification, wherein the second compound has a structure represented by formula (IV): 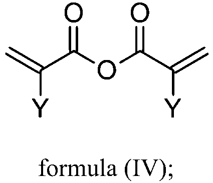
performing a washing step, wherein the product for purification is precipitated by and washed with an alcohol solvent to form a product for drying; and
performing a drying step, wherein the product for drying is filtered and dried to obtain the polycarbonate oligomer with unsaturated double bonds;
wherein R₁ is a hydrogen atom, an alkyl group with a carbon number of 1 to 6, an allyl group, an alkoxy group with a carbon number of 1 to 6, an aryl group with a carbon number of 6 to 12, or a halogen atom; R₂ is an alkyl group with a carbon number of 1 to 12; X is a single bond, a cycloalkyl group with a carbon number of 3 to 12, a structure represented by formula (1), formula (2), formula (3), formula (4), formula (5), formula (6), formula (7), formula (8), or formula (9): 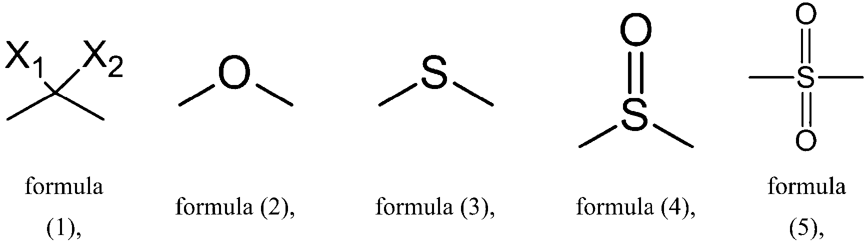 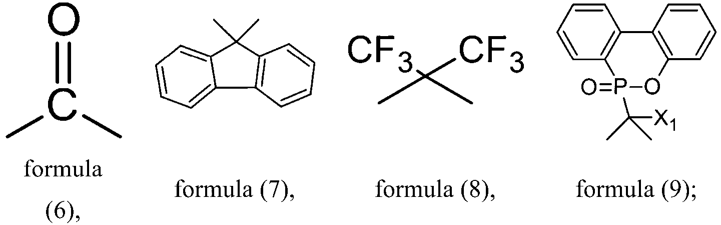
wherein X₁ and X₂ are a hydrogen atom, an alkyl group with a carbon number of 1 to 6, or an aryl group with a carbon number of 6 to 12, independently;
wherein Y is a hydrogen atom or a methyl group, a is an integer of 0 to 4, and n is an arbitrary number of 0 to 30.

3. The manufacturing method of claim 2, **characterized in that** a molar ratio of the polycarbonate to the first compound is 1:10 to 1:50.

4. The manufacturing method of claim 2, **characterized in that** the reaction solvent is selected from the group consisting of N,N-dimethylacetamide, N-methylpyrrolidone, dimethylformamide, methoxybenzene, dimethyl sulfoxide, propylene glycol methyl ether acetate, propylene glycol methyl ether propionate and cyclohexanone.

5. The manufacturing method of claim 2, **characterized in that** the first catalyst and second catalyst are respectively selected from the group consisting of 4-dimethylaminopyridine, imidazole, 2-methylimidazole, 2-phenylimidazole, 2-ethyl-4-methylimidazole, 1,8-diazabicyclo[5.4.0]undec-7-ene, and 1,5,7-triazabicyclo[4.4.0]dec-5-ene.

6. The manufacturing method of claim 2, **characterized in that** a molar ratio of the first compound to the first catalyst is 1:0.002 to 1:0.004.

7. The manufacturing method of claim 2, **characterized in that**, in the alcoholysis step, the alcoholysis reaction between the polycarbonate and the first compound is conducted at 70 °C to 180 °C.

8. The manufacturing method of claim 2, **characterized in that** an addition amount of the first catalyst and the second catalyst is 10 weight percent to 30 weight percent of a content of the first compound.

9. The manufacturing method of claim 2, **characterized in that** a molar ratio of the first compound to the second compound is 1:1 to 1:5.

10. The manufacturing method of claim 2, **characterized in that**, in the esterification step, the esterification reaction between the polycarbonate oligomer with the one-sided unsaturated double bond and the second compound is conducted at 70 °C to 100 °C.

11. A curable composition, **characterized in that** the curable composition comprises the polycarbonate oligomer with unsaturated double bonds of claim 1, a polyphenylene ether resin with unsaturated double bonds, a solvent, and a free radical initiator.

12. The curable composition of claim 11, **characterized in that** a mass ratio of the polycarbonate oligomer with unsaturated double bonds to the polyphenylene ether resin with unsaturated double bonds is 0.1 to 1.

13. The curable composition of claim 11, **characterized in that** the free radical initiator is selected from the group consisting of a peroxide and an azo initiator.

14. The curable composition of claim 11, **characterized in that** an addition amount of the free radical initiator is 0.5 weight percent to 3.0 weight percent of a total content of the polycarbonate oligomer with unsaturated double bonds and the polyphenylene ether resin with unsaturated double bonds.

15. The curable composition of claim 11, **characterized in that** the solvent is selected from the group consisting of N,N-dimethylacetamide, N-methylpyrrolidone, dimethylformamide, methoxybenzene, dimethyl sulfoxide, propylene glycol methyl ether acetate, propylene glycol methyl ether propionate, γ-butyrolactone, butanone, cyclohexanone, toluene and xylene.

16. A manufacturing method of a cured product with low dielectric property, **characterized in that** the curable composition of claim 11 is cured at a curing temperature to obtain a cured product with low dielectric property, wherein the curing temperature is 140 °C to 250 °C.
